# FASCICULE DE BREVET EUROPEEN

(11) **EP 0 723 972 B1**
(45) Date de publication et mention de la délivrance du brevet: **25.07.2001**
(21) Numéro de dépôt: 96420032.3
(22) Date de dépôt: 30.01.1996
(51) Int. Cl.: C07H 15/04, A61K 31/70

(54) **Lactylamines et applications pharmaceutiques**
Lactyl-aminen und ihre pharmazeutische Verwendung
Lactylamines and their pharmaceutical application

(30) Priorité: 30.01.1995 FR 9501296
(43) Date de publication de la demande: 31.07.1996
(73) Titulaire: STEPAN EUROPE, 38340 Voreppe (FR)
(72) Inventeur: Rico-Lattes, Isabelle, F-31650 Auzielle (FR); Lattes, Armand, F-31650 Auzielle (FR); Caparros, Alain, F-31600 Muret (FR); Andre-Barres, Christiane, F-31500 Toulouse (FR); Godefroy, Lionel, F-38430 Moirans (FR)
(74) Mandataire: Guerre, Dominique

(56) Documents cités:
- FR-A- 2 661 413
- CARBOHYDRATE RESEARCH, vol. 74, 1979, AMSTERDAM, pages 135-143, XP002000508 P.D.HOAGLAND ET AL.: "Reductive Amination of Lactose : Unusual 13C-N.M.R. Spectroscopic Properties of N-alkyl-(1-deoxylactitol-1-yl)amines"
- BIOCHIMICA ET BIOPHYSICA ACTA, vol. 1109, 1992, pages 55-58, XP002000509 R.GARELLI-CALVET ET AL.: "A New Surfactant Series, the N-Alkylamino-1-deoxylactitols : Application for Extraction of 'op' Opiate Receptors from Frog Brain"
- JOURNAL OF DISPERSION TECHNOLOGY, vol. 12, no. 3&4, 1991, pages 227-237, XP002000510 P.LATGE ET AL.: "Synthesis of Long Chain N-allyllactylamines from Unprotected Lactose A New Series of Non-ionic Surfactants"
- NEW JOURNAL OF CHEMISTRY, vol. 19, 1995, pages 341-344, XP002000511 I.RICO-LATTES : "A Short Route to Analogs of Galactosphingophospholipids possessing Anti-HIV Activity and Anti-Aspergillus Activity."

## Description

La présente invention concerne des lactylamines, leurs applications dans le domaine pharmaceutique et un procédé pour leur préparation.

Selon la demande de brevet FR-A-2 661 413 au nom de la Demanderesse, il est décrit des N-alkyllactylamines dont la structure répond à la formule I : 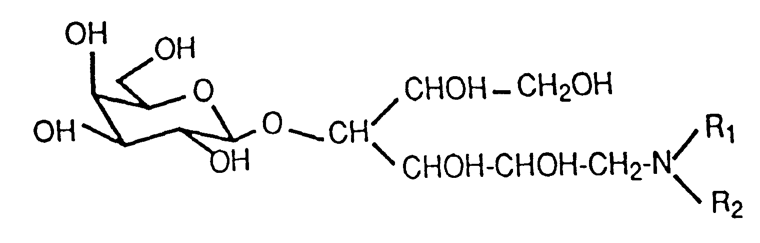 dans laquelle R₁ représente -(CH₂)ₙ-CH₃, n étant compris entre 0 et 19, et R₂ représente H.

Les N-alkylactyllamines (appelées composés **A1**) révélées par cet art antérieur sont très stables en solution aqueuse et possèdent des propriétés tensioactives.

Ce document décrit en outre un procédé pour préparer ces N-alkyllactylamines à partir de N-alkyllactosylamines que l'on soumet à une étape de réduction par action d'un hydrure métallique ou par réduction catalytique.

Au début du document, sont évoquées des N-alkyl-N-acyl-lactylamines dont la structure répond à la formule I mentionnée ci-dessus, dans laquelle R₁ a la même définition que précédemment, et R₂ représente un groupement acyle ayant de 2 à 10 atomes de carbone. Néanmoins, l'enseignement de ce document est insuffisant pour permettre à l'homme du métier d'accéder à de tels composés. En effet, si les conditions de réaction de la réduction des N-alkylactylamines sont détaillées, celles conduisant à la réduction sélective du lactose et non du groupement acyle pour le cas des N-alkyl-N-acyllactylamines, sont absentes.

Un premier objet de l'invention est une famille de composés dérivés des lactylamines dont la structure répond à la formule I, dans laquelle :
- R₁ représente -(CH₂)ₙ-CH₃ ou -(CH₂)ₙ-COO⁻X⁺,
   n étant compris entre 5 et 15,
   X⁺ représentant un cation notamment choisi parmi H⁺, Na⁺, K⁺, NH₄⁺, Li⁺, Mg²⁺,
- R₂ CO-(CH₂)ₘ-CH_{3,}
   m étant compris entre 5 et 16.

De manière totalement inattendue, la Demanderesse a découvert que les composés de la présente invention décrits ci-dessus, ainsi que les N-alkyllactylamines révélées par FR-A-2 661 413, constituent des analogues structuraux du galactosyl-céramide Gal-β₁-Cer possédant la structure de formule **II** suivante :

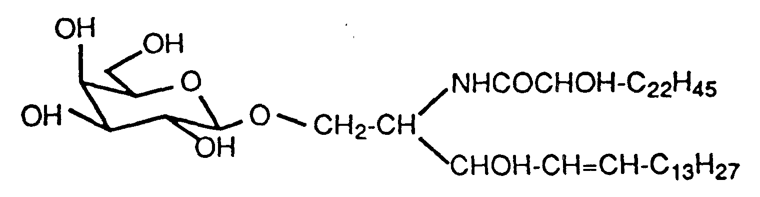

Ce glycosphingolipide a été identifié comme un site récepteur potentiel de la glycoprotéine externe d'enveloppe gp120 du VIH, dans deux types cellulaires différents : les oligodendrocytes du cerveau **(1)** et les cellules épithéliales de la muqueuse intestinale **(2)**.

Ce comportement d'analogue strutural des composés précités est d'autant plus surprenant, que ledit site récepteur Gal-β₁-Cer est très spécifique de la protéine gp120, puisque cette dernière ne s'associe pas aux analogues glucosyl-céramides ou lactosyl-céramides **(4)**.

La Demanderesse a aussi découvert que les composés précités présentent une activité anti-Aspergillus fumigatus. L'infection par ce champignon engendre des mycoses systémiques, et se rencontre de plus en plus fréquemment en particulier chez les sujets immunodéprimés, notamment atteints du SIDA.

Les propriétés anti-VIH des composés précités, ainsi que les propriétés anti-Aspergillus fumigatus, ont pu être mises en évidence au cours de tests spécifiques standardisés, abordés plus loin dans la description.

Ainsi, l'invention concerne les applications d'un composé dont la structure répond à la formule I : 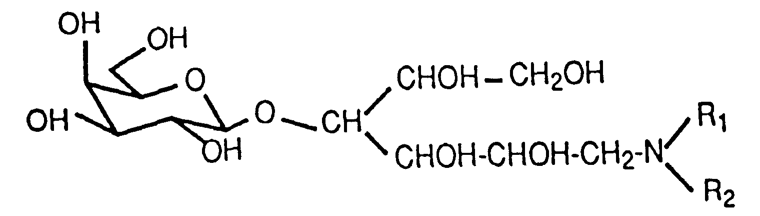 dans laquelle :
- R₁ représente -(CH₂)ₙ-CH₃ ou -(CH₂)ₙ-COO⁻X⁺,
   n étant compris entre 5 et 15,
   X⁺ représentant un cation notamment choisi parmi H⁺, Na⁺, K⁺, NH₄⁺, Li⁺, Mg²⁺, et
- R₂ CO-(CH₂)ₘ-CH₃,
   m étant compris entre 5 et 16,
ou les applications d'un sel pharmaceutiquement acceptable dudit composé, en tant que médicament ou introduit à titre de principe actif, dans une composition médicamenteuse ou prophylactique ou thérapeutique, ledit composé ou sel étant éventuellement associé à un autre principe actif, et étant combiné à un support pharmaceutiquement acceptable.

Un autre objet de l'invention est un agent pour prévenir ou traiter une infection par le VIH comprenant un composé décrit ci-dessus ou un sel pharmaceutiquement acceptable dudit composé, et un agent pour prévenir ou traiter une infection par Aspergillus fumigatus.

Par ailleurs, la Demanderesse a mis au point un procédé de préparation des composés de l'invention qui est doublement avantageux : d'une part la matière première est le lactose, un disaccharide peu coûteux et facilement isolé, et d'autre part le nombre d'étapes permettant d'accéder aux composés de l'invention à partir du lactose est très limité :
- deux étapes seulement sont nécessaires pour obtenir un composé de formule **I** dans lequel R₁ représente -(CH₂)ₙ-COO⁻X⁺ et R₂ représente H, dénommé composé **A2;**
- trois sont nécessaires pour obtenir un composé de formule **I** dans lequel R₁ représente CH₃-(CH₂)ₙ (composé **B1**) ou (CH₂)ₙCOO⁻X⁺ (composé **B2**) et R₂ représente CO-(CH₂)ₘ-CH₃.

Pour synthétiser les composés **A2,** on met en réaction du lactose avec un amino-acide de formule **III**, NH₂-(CH₂)ₙ-COOH, en présence d'une base, puis on réduit la N-acyl-lactosylamine obtenue.

Les composés **B1** et **B2** peuvent être obtenus à partir des composés respectivement **A1** et **A2** que l'on met en réaction avec un agent acylant, conduisant à une acylation sélective des fonctions amines, sans réagir avec les groupements hydroxyle **(3)**. Cet agent acylant répond à la formule **IV**, R₂-CO-Z dans laquelle Z représente notamment : 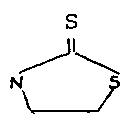 et R₂ représente CO-(CH₂)ₘ-CH₃, m étant compris entre 5 et 16.

L'étape de réduction de la N-acyl-lactosylamine en composé **A2** conduit spécifiquement à la liaison β₁-glycosidique entre le galactose et la partie aminée indispensable à la reconnaissance par la protéine gp120.

Préférentiellement, cette étape de réduction est effectuée par le NaBH₄.

Les différents objets de l'invention sont à présent illustrés avec les Exemples 1 à 15 suivants.

### EXEMPLE 1: Synthèse de A2, avec R₁=-(CH₂)₇-COO⁻NH4⁺

A une solution du ω-amino-acide NH₂-(CH₂)₇-CO₂H (5,5 mmoles) et de soude (5,5 mmoles) dans 100 ml de méthanol, sont ajoutées 5,5 mmoles de lactose dissoutes dans 40 ml d'eau. L'ensemble est mis sous agitation pendant 24 h à 40°C. 10 mmoles de NaBH₄ sont ensuite additionnées au mélange. L'ensemble est mis sous agitation 4 h de plus à température ambiante.

Après évaporation du solvant, le composé est purifié sur colonne de silice (éluant: CHCl₃ = 1,25 / MeOH = 7 / ammoniaque = 1,75, en volume).

Le composé **A2** est ainsi obtenu avec un rendement de 30% sous forme d'une fine poudre blanche après lyophilisation. Il est caractérisé par RMN¹H, ¹³C, spectrométrie de masse et microanalyse.

### EXEMPLE 2: Synthèse de B2_{(8, 11)}, avec R₁=-(CH₂)₇-COO⁻NH₄⁺ et R₂=-CO-(CH₂)₁₀-CH₃

A une solution de 1,77 mmole de composé **A2,** obtenu dans l'Exemple 1, dans 100 ml de DMF, sont ajoutées goutte à goutte 2,65 mmoles de réactif acylant, de formule **IV**: 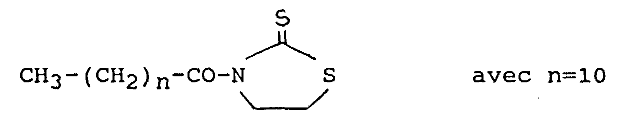 préparé aisément en deux étapes à partir de la mercaptothiazoline et le chlorure d'acide C₁₁H₂₃-COCl **(7)**. Le mélange est laissé sous agitation pendant 5 jours à 55°C. Après évaporation le composé **B2**_{**(8, 11)**} est purifié sur colonne de silice (éluant = CHCl₃ / MeOH / NH₄OH = 7/2,2/0,5, en volume).

Le composé **B2**_{**(8, 11)**} est obtenu avec un rendement de 55% sous forme d'une poudre blanche après lyophilisation. Il est caractérisé par RMN¹H, ¹³C, spectrométrie de masse et microanalyse.

### EXEMPLE 3: Synthèse de B1_{(12, 11)}, avec R₁=-(CH₂)₁₁-CH₃ et R₂=-CO-(CH₂)₁₀-CH₃

A une solution de 1 g de N-dodécylaminodésoxy-1-lactitol précédemment décrit **(6)** dans 30 ml de DMF, on ajoute 0,589 g de réactif acylant, de formule **IV**:

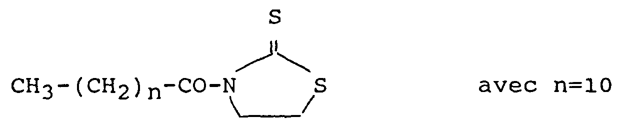

Le mélange est chauffé sous agitation à 70°C pendant 6 jours. Le DMF est évaporé. Le résidu est purifié sur colonne de silice (éluant = CHCl₃ / MeOH / NH₄OH = 7/2,5/0,5, en volume).

Le composé **B1**_{**(12, 11)**} est obtenu avec un rendement de 53% sous forme d'une poudre blanche après lyophilisation. Il est caractérisé par RMN¹H, ¹³C, spectrométrie de masse et microanalyse.

### EXEMPLE 4: Synthèse de B1_{(9, 17)}, avec R₁=-(CH₂)₈-CH₃ et R₂=-CO-(CH₂)₁₆-CH₃

On opère comme dans l'Exemple 3 à partir de 1 g de N-nonylaminodésoxy-1-lactitol et de 3,28 g du réactif acylant, dans la formule **IV** duquel n=16.

Après traitement analogue à celui de l'Exemple 3, on récupère le composé **B1**_{**(9, 17)**} avec un rendement de 43% sous forme de poudre blanche.

### EXEMPLE 5: Synthèse de B1_{(9, 11)}, avec R₁=-(CH₂)₈-CH₃ et R₂=-CO-(CH₂)₁₀-CH₃

On opère comme dans l'Exemple 3 à partir de 1 g de N-nonylaminodésoxy-1-lactitol et de 0,64 g du réactif acylant, dans la formule **IV** duquel n=10.

Après traitement analogue à celui de l'Exemple 3, on récupère le composé **B1**_{**(9, 11)**} sous forme d'un solide blanc avec un rendement de 63%.

### EXEMPLE 6: Synthèse de B1_{(9, 15)}, avec R₁=-(CH₂)₈-CH₃ et R₂=-CO-(CH₂)₁₄-CH₃

On opère comme dans l'Exemple 3 à partir de 1 g de N-nonylaminodésoxy-1-lactitol et de 0,749 g du réactif acylant, dans la formule **IV** duquel n=14.

Après traitement analogue à celui de l'Exemple 3, on récupère le composé **B1**_{**(9, 15)**} sous forme d'un solide blanc avec un rendement de 21%.

### EXEMPLE 7: Synthèse de B1_{(9, 13)}, avec R₁=-(CH₂)₈-CH₃ et R₂=-CO-(CH₂)₁₂-CH₃

On opère comme dans l'Exemple 3 à partir de 4,98 g de N-nonylaminodésoxy-1-lactitol et de 2,75 g du réactif acylant, dans la formule **IV** duquel n=12.

Après traitement analogue à celui de l'Exemple 3, on récupère le composé **B1**_{**(9, 13)**} sous forme d'une poudre blanche avec un rendement de 51%.

### EXEMPLE 8: Synthèse de B1_{(9, 8)}, avec R₁=-(CH₂)₈-CH₃ et R₂=-CO-(CH₂)₇-CH₃

A partir de 0,7 g de N-nonylaminodésoxy-1-lactitol et de 0,463 g du réactif acylant, dans la formule **IV** duquel n=7, on opère comme dans l'Exemple 3, on récupère le composé **B1**_{**(9, 8)**} sous forme d'une poudre blanche avec un rendement de 15%.

### EXEMPLE 9: Synthèse de B1_{(6, 6)}, avec R₁=-(CH₂)₅-CH₃ et R₂=-CO-(CH₂)₅-CH₃

A partir de 0,4 g de N-hexylaminodésoxy-1-lactitol et de 0,20 g du réactif acylant, dans la formule **IV** duquel n=5, on opère comme dans l'Exemple 3, on récupère le composé **B1**_{**(6, 6)**} sous forme d'une poudre blanche avec un rendement de 20%.

### EXEMPLE 10:

Les composés **B (B1, B2)** sont testés du point de vue de leurs propriétés anti-VIH selon un protocole d'essai in vitro standardisé et complètement décrit dans les publications **(5, 6)**, dont le contenu est incorporé par référence, à la présente description.

La multiplication du VIH (souche BRU) dans les cellules CEM est évaluée par le nombre de syncytia formés après 4 jours d'infection, résultant de l'interaction entre la glycoprotéine gp120 exprimée par les cellules (BRU) infectées par le VIH, et les lymphocytes T4 (CEM).

Les composés testés sont ajoutés après l'adsorption du virus dans le milieu de culture aux concentrations finales 10⁻³M, 10⁻⁴M, 10⁻⁵M, 10⁻⁶M et 10⁻⁷M.

Les résultats sont exprimés par la concentration la plus faible de composés qui diminue la formation des syncytia d'au moins 50%. Cette concentration est appelée EC₅₀. Elle doit être la plus basse possible.

On détermine aussi la toxicité des composés testés. L'effet toxique sur les CEM non infectées est apprécié par une réaction colorimétrique basée sur la capacité des cellules vivantes à réduire le bromure de 3-(4,5-diméthylthiazol-2-yl)-2,5-diphényltétrazolium en formazan après quatre jours d'incubation en présence de différentes concentrations des composés. Les résultats sont exprimés par la concentration la plus faible de composés qui provoque une inhibition d'au moins 50% de la formation de formazan. Cette concentration est appelée CI₅₀.

Les composés **B** se révèlent au cours de ces tests, hautement actifs contre le VIH.

### EXEMPLE 11:

Le composé **B1(**_{**9, 15**}**)** synthétisé dans l'Exemple 6, présente une EC₅₀ de 10 *µ*M et une CI₅₀ supérieure à 100 *µ*M quand il est testé comme anti-VIH sur macrophage CEM-SS.

### EXEMPLE 12:

Différents composés **B1** ont été testés du point de vue de leurs propriétés anti*-Aspergillus fumigatus*, dans un essai in vitro standardisé, complètement décrit dans la publication **(7)** dont le contenu est incorporé, par référence, à la présente description.

Dans cet essai, la croissance dudit champignon est indiquée par la consommation de glucose qui constitue la seule source de carbone métabolisable, et on mesure la concentration en composé testé nécessaire pour inhiber 50% (IC₅₀), et 90% (IC₉₀) de la croissance du champignon. Deux composés témoin sont utilisés, l'Itraconazole et l'Amphotéricine B, dont les propriétés anti-*Aspergillus fumigatus* sont connues et qui servent dans cet essai de référence.

Les résultats sont présentés dans le Tableau I suivant :

**TABLEAU I**

| Composés | CI₉₀ | CI₉₀ | CI₅₀ | CI₅₀ |
|---|---|---|---|---|
| | µg/ml | µmole/l | µg/ml | µmole/l |
| Itraconazole | 0.06 | 0.08 | 0.003 | 0.004 |
| Amphotéricine B | 0.6 | 0.6 | 0.02 | 0.02 |
| **B**_{**(9, 13)**} | 18.0 | 26,5 | 5.0 | 7.4 |
| **B**_{**(9, 15)**} | 7.0 | 9.8 | 1,6 | 2.3 |
| **B**_{**(9, 17)**} | 1.8 | 2.4 | 0,5 | 0,6 |

Ces résultats mettent notamment en évidence l'intérêt particulier du composé **B**_{**(9, 17)**}**,** dont l'activité est proche de celle de l'Amphotéricine B.

### REFERENCES

(1) F. GONZALES-SCARANO et coll. Science, 1991, 253, 320
(2) J. FANTINI et coll. J. Virol 1992, 66, 4848
(3) I. RICO et coll. Synth. Comm., 1993, 23, 949
(4) J. FANTINI et coll. Medecine/Science, 1993, 9, 891
(5) R. PAUWELS, J. Virol, Methods, 1988, 20, 309
(6) D. SCHOLS, J. Gen. Virol, 1989, 70, 2397
(7) J.C. GARRIGUES, G. CADET DE FONTENAY, M.D. LINAS, M. LAGENTE et J.P. SEGUELA, Antimicrob. Agents Chemoth, 1994, 38, 2857

## Revendications

1. Composés dont la structure répond à la formule **I** : 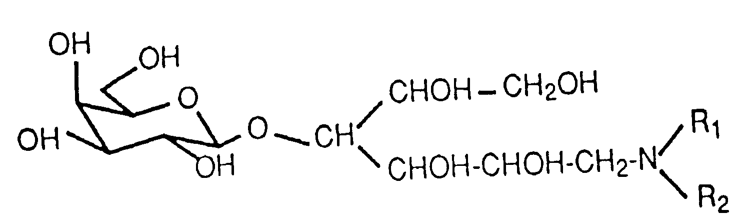 dans laquelle :
- R₁ représente -(CH₂)ₙ-CH₃ ou -(CH₂)ₙ-COO⁻X⁺,
n étant compris entre 5 et 15,
X⁺ représentant un cation notamment choisi parmi H⁺, Na⁺, K⁺, NH₄⁺, Li⁺, Mg²⁺, et
- R₂ représente CO-(CH₂)ₘ-CH₃,
m étant compris entre 5 et 16.

2. Médicament comprenant un composé dont la structure répond à la formule **I** : 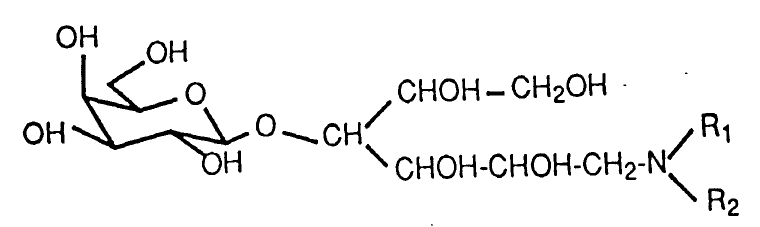 dans laquelle :
- R₁ représente -(CH₂)ₙ-CH₃ ou -(CH₂)ₙ-COO⁻X⁺,
n étant compris entre 5 et 15,
X⁺ représentant un cation notamment choisi parmi H⁺, Na⁺, K⁺, NH₄⁺, Li⁺, Mg²⁺, et
- R₂ représente CO-(CH₂)ₘ-CH₃,
m étant compris entre 5 et 16,
ou comprenant un sel pharmaceutiquement acceptable dudit composé.

3. Utilisation d'un composé dont la structure répond à la formule **I** : 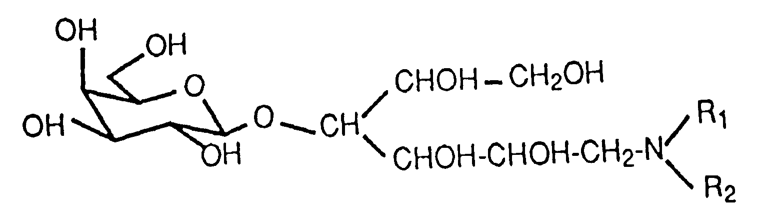 dans laquelle :
- R₁ représente -(CH₂)ₙ-CH₃ ou -(CH₂)ₙ-COO⁻X⁺,
n étant compris entre 5 et 15,
X⁺ représentant un cation notamment choisi parmi H⁺, Na⁺, K⁺, NH₄⁺, Li⁺, Mg²⁺, et
- R₂ représente CO-(CH₂)ₘ-CH₃,
m étant compris entre 5 et 16,
ou des sels pharmaceutiquement acceptables dudit composé, pour l'obtention d'un médicament destiné à la prévention ou traitement d'une infection par Aspergillus fumigatus.

4. Composition médicamenteuse prophylactique ou thérapeutique comprenant, à titre de principe actif, un composé dont la structure répond à la formule **I** : 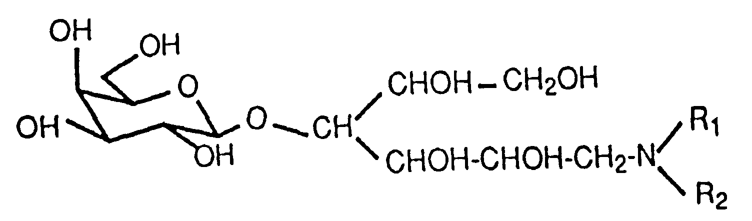 dans laquelle :
- R₁ représente -(CH₂)ₙ-CH₃ ou -(CH₂)ₙ-COO⁻X⁺,
n étant compris entre 5 et 15,
X⁺ représentant un cation notamment choisi parmi H⁺, Na⁺, K⁺, NH₄⁺, Li⁺, Mg²⁺, et
- R₂ représente CO-(CH₂)ₘ-CH₃,
m étant compris entre 5 et 16,
ou un sel pharmaceutiquement acceptable dudit composé, éventuellement associé à un autre principe actif, et combiné à un support pharmaceutiquement acceptable.

5. Agent pour prévenir ou traiter une infection par le VIH-I comprenant un composé dont la structure répond à la formule **I** : 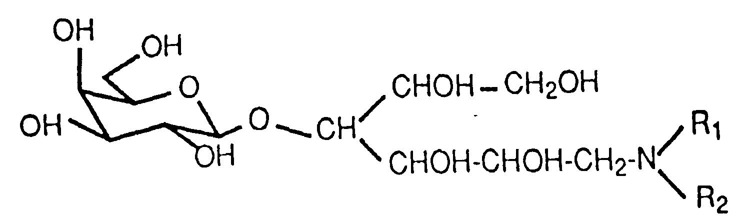 dans laquelle :
- R₁ représente-(CH₂)ₙ-CH₃ ou -(CH₂)ₙ-COO⁻X⁺,
n étant compris entre 5 et 15,
X⁺ représentant un cation notamment choisi parmi H⁺, Na⁺, K⁺, NH₄⁺, Li⁺, Mg²⁺, et
- R₂ représente CO-(CH₂)ₘ-CH₃,
m étant compris entre 5 et 16,
ou un sel pharmaceutiquement acceptable dudit composé.

6. Agent pour prévenir ou traiter une infection par Aspergillus fumigatus comprenant un composé dont la structure répond à la formule **I** : 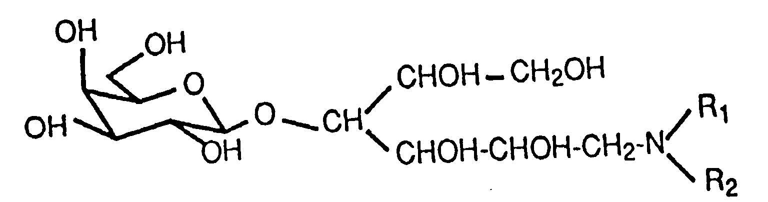 dans laquelle :
- R₁ représente -(CH₂)ₙ-CH₃ ou -(CH₂)ₙ-COO⁻X⁺,
n étant compris entre 5 et 15,
X⁺ représentant un cation notamment choisi parmi H⁺, Na⁺, K⁺, NH₄⁺, Li⁺, Mg²⁺, et
- R₂ représente CO-(CH₂)ₘ-CH₃,
m étant compris entre 5 et 16,
ou un sel pharmaceutiquement acceptable dudit composé.

7. Utilisation d'un composé dont la structure répond à la formule **I** : 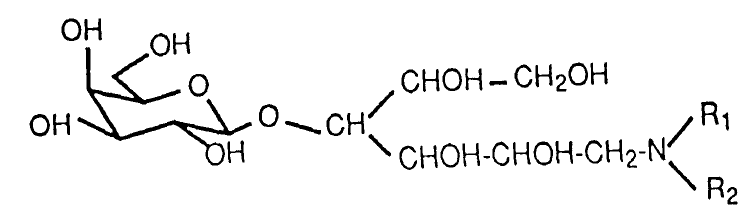 dans laquelle :
- R₁ représente -(CH₂)ₙ-CH₃ ou -(CH₂)ₙ-COO⁻X⁺,
n étant compris entre 5 et 15,
X⁺ représentant un cation notamment choisi parmi H⁺, Na⁺, K⁺, NH₄⁺, Li⁺, Mg²⁺, et
- R₂ représente CO-(CH₂)ₘ-CH₃,
m étant compris entre 5 et 16,
ou d'un sel pharmaceutiquement acceptable, pour préparer un médicament destiné à prévenir ou traiter une infection par le VIH-I.

8. Procédé pour préparer des composés dont la structure répond à la formule **I**: 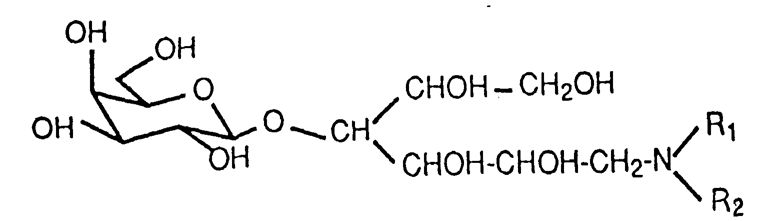 dans laquelle :
- R₁ représente -(CH₂)ₙ-CH₃ ou -(CH₂)ₙ-COO⁻X⁺,
n étant compris entre 5 et 15,
X⁺ représentant un cation notamment choisi parmi H⁺, Na⁺, K⁺, NH₄⁺, Li⁺, Mg²⁺, et
- R₂ représente CO-(CH₂)ₘ-CH₃,
m étant compris entre 5 et 16,
caractérisé en ce qu'on met en réaction le composé répondant à la formule 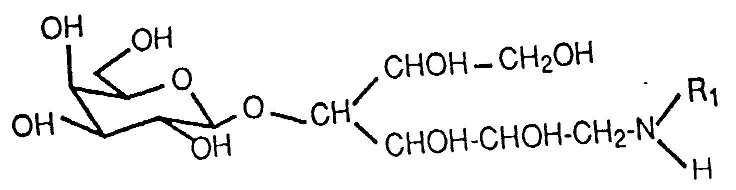 dans laquelle :
- R₁ représente -(CH₂)ₙ-CH₃ ou -(CH₂)ₙ-COO⁻X⁺,
n étant compris entre 5 et 15,
X⁺ représentant un cation notamment choisi parmi H⁺, Na⁺, K⁺, NH₄⁺, Li⁺, Mg²⁺,
avec un agent acylant ayant la formule **IV,** R₂-CO-Z, dans laquelle Z représente notamment 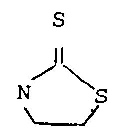 et R₂ représente CH₃-(CH₂)ₘ-CO, m étant compris entre 5 et 16.

9. Procédé pour préparer des composés dont la structure répond à la formule **I**: 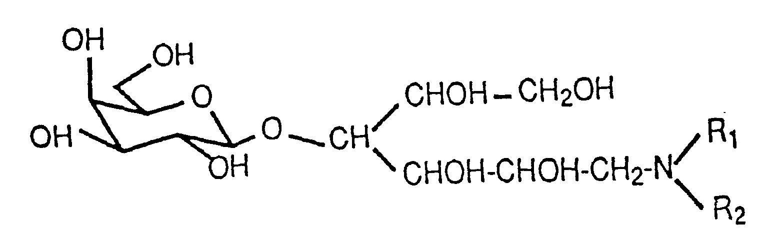 dans laquelle :
- R₁ représente -(CH₂)ₙ-CH₃ ou -(CH₂)ₙ-COO⁻X⁺,
n étant compris entre 5 et 15,
X⁺ représentant un cation notamment choisi parmi H⁺, Na⁺, K⁺, NH₄⁺, Li⁺, Mg²⁺, et
- R₂ représente CO-(CH₂)ₘ-CH₃,
m étant compris entre 5 et 16,
caractérisé en ce qu'on met en réaction du lactose avec un amino-acide de formule **III**, NH₂-(CH₂)ₙ-COOH dans laquelle n a la définition ci-dessus, en présence d'une base, puis on réduit la N-acyl-lactosylamine obtenue,
puis qu'on met en réaction le composé ainsi obtenu répondant à la formule 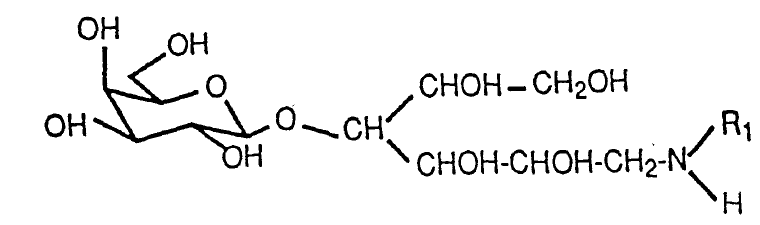 dans laquelle :
- R₁ représente -(CH₂)ₙ-CH₃ ou -(CH₂)ₙ-COO⁻X⁺,
n étant compris entre 5 et 15,
X⁺ représentant un cation notamment choisi parmi H⁺, Na⁺, K⁺, NH₄⁺, Li⁺, Mg²⁺,
avec un agent acylant ayant la formule **IV,** R₂-CO-Z, dans laquelle Z représente notamment 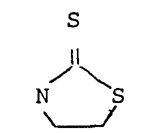 et R₂ représente CH₃-(CH₂)ₘ-CO, m étant compris entre 5 et 16.

10. Procédé selon la revendication 9, caractérisé en ce que l'étape de réduction est effectuée avec du NaBH₄.

## Patentansprüche

1. Verbindungen, deren Struktur der Formel I entspricht: 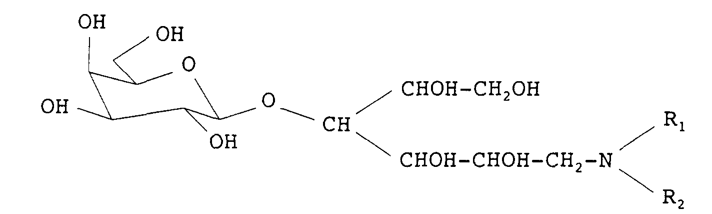 in der:
- R₁ für -(CH₂)ₙ-CH₃ oder -(CH₂)ₙ-COO⁻X⁺ steht, wobei
n bei 5 bis 15 liegt,
X⁺ für ein Kation steht, insbesondere ausgewählt aus H⁺, Na⁺, K⁺, NH₄⁺, Li⁺, Mg²⁺, und
- R₂ für CO-(CH₂)ₘ-CH₃ steht, wobei
m bei 5 bis 16 liegt.

2. Arzneimittel, das eine Verbindung aufweist, deren Struktur der Formel I entspricht: 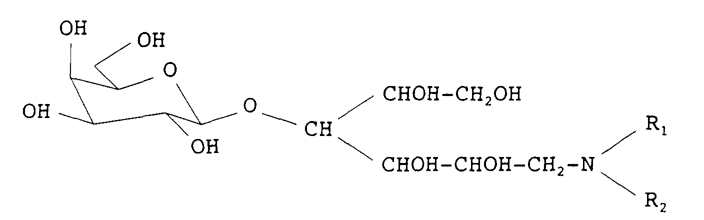 in der:
- R₁ für -(CH₂)ₙ-CH₃ oder -(CH₂)ₙ-COO⁻X⁺ steht, wobei
n bei 5 bis 15 liegt,
X⁺ für ein Kation steht, insbesondere ausgewählt aus H⁺, Na⁺, K⁺, NH₄⁺, Li⁺, Mg²⁺, und
- R₂ für CO-(CH₂)ₘ-CH₃ steht, wobei
m bei 5 bis 16 liegt,
oder das ein pharmazeutisch akzeptables Salz dieser Verbindung aufweist.

3. Verwendung von einer Verbindung, deren Struktur der Formel I entspricht: 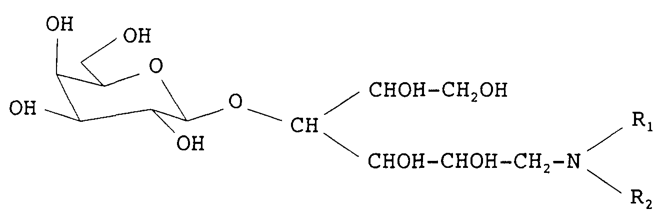 in der:
- R₁ für -(CH₂)ₙ-CH₃ oder -(CH₂)ₙ-COO⁻X⁺ steht, wobei
n bei 5 bis 15 liegt,
X⁺ für ein Kation steht, insbesondere ausgewählt aus H⁺, Na⁺, K⁺, NH₄⁺, Li⁺, Mg²⁺, und
- R₂ für CO-(CH₂)ₘ-CH₃ steht, wobei
m bei 5 bis 16 liegt,
oder von pharmazeutisch akzeptablen Salzen dieser Verbindung, um ein Arzneimittel herzustellen, das für die Prävention oder Behandlung einer Infektion durch Aspergillus fumigatus bestimmt ist.

4. Prophylaktische oder therapeutische medikamentöse Zusammensetzung, die als Wirkstoff eine Verbindung aufweist, deren Struktur der Formel I entspricht: 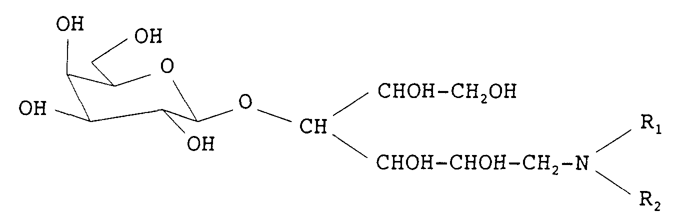 in der:
- R₁ für -(CH₂)ₙ-CH₃ oder -(CH₂)ₙ-COO⁻X⁺ steht, wobei
n bei 5 bis 15 liegt,
X⁺ für ein Kation steht, insbesondere ausgewählt aus H⁺, Na⁺, K⁺, NH₄⁺, Li⁺, Mg²⁺, und
- R₂ für CO-(CH₂)ₘ-CH₃ steht, wobei
m bei 5 bis 16 liegt,
oder ein pharmazeutisch akzeptables Salz dieser Verbindung, das ggf. mit einem anderen Wirkstoff assoziiert ist, und welche mit einem pharmazeutisch akzeptablen Träger verbunden ist.

5. Agens zum Vorbeugen oder Behandeln einer Infektion durch HIV-I, das eine Verbindung aufweist, deren Struktur der Formel I entspricht: 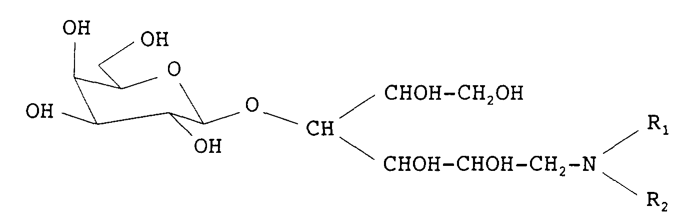 in der:
- R₁ für -(CH₂)ₙ-CH₃ oder-(CH₂)ₙ-COO⁻X⁺ steht, wobei
n bei 5 bis 15 liegt,
X⁺ für ein Kation steht, insbesondere ausgewählt aus H⁺, Na⁺, K⁺, NH₄⁺, Li⁺, Mg²⁺, und
X⁺ für ein Kation steht, insbesondere ausgewählt aus H⁺, Na⁺, K⁺, NH₄⁺, Li⁺, Mg²⁺, und
- R₂ für CO-(CH₂)ₘ-CH₃ steht, wobei
m bei 5 bis 16 liegt,
oder ein pharmazeutisch akzeptables Salz dieser Verbindung.

6. Agens zum Vorbeugen oder Behandeln einer Infektion durch Aspergillus fumigatus, das eine Verbindung aufweist, deren Struktur der Formel I entspricht: 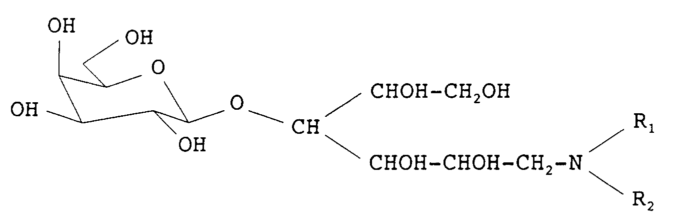 in der:
- R₁ für -(CH₂)ₙ-CH₃ oder -(CH₂)ₙ-COO⁻X⁺ steht, wobei
n bei 5 bis 15 liegt,
X⁺ für ein Kation steht, insbesondere ausgewählt aus H⁺, Na⁺, K⁺, NH₄⁺, Li⁺, Mg²⁺, und
- R₂ für CO-(CH₂)ₘ-CH₃ steht, wobei
m bei 5 bis 16 liegt,
oder ein pharmazeutisch akzeptables Salz dieser Verbindung.

7. Verwendung einer Verbindung, deren Struktur der Formel I entspricht: 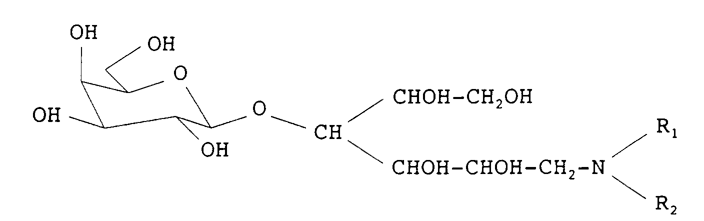 in der:
- R₁ für -(CH₂)ₙ-CH₃ oder -(CH₂)ₙ-COO⁻X⁺ steht, wobei
n bei 5 bis 15 liegt,
X⁺ für ein Kation steht, insbesondere ausgewählt aus H⁺, Na⁺, K⁺, NH₄⁺, Li⁺, Mg²⁺, und
- R₂ für CO-(CH₂)ₘ-CH₃ steht, wobei
m bei 5 bis 16 liegt,
oder von einem pharmazeutisch akzeptablen Salz, zur Herstellung eines Arzneimittels, das zur Vorbeugung oder Behandlung einer Infektion durch HIV-I bestimmt ist.

8. Verfahren zum Herstellen von Verbindungen, deren Struktur der Formel I entspricht: 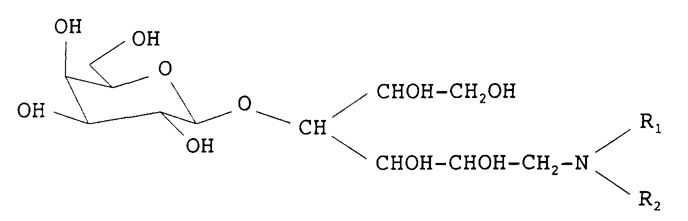 in der:
- R₁ für -(CH₂)ₙ-CH₃ oder -(CH₂)ₙ-COO⁻X⁺ steht, wobei
n bei 5 bis 15 liegt,
X⁺ für ein Kation steht, insbesondere ausgewählt aus H⁺, Na⁺, K⁺, NH₄⁺, Li⁺, Mg²⁺, und
- R₂ für CO-(CH₂)ₘ-CH₃ steht, wobei
m bei 5 bis 16 liegt,
dadurch gekennzeichnet, daß man die Verbindung, die der Formel entspricht, 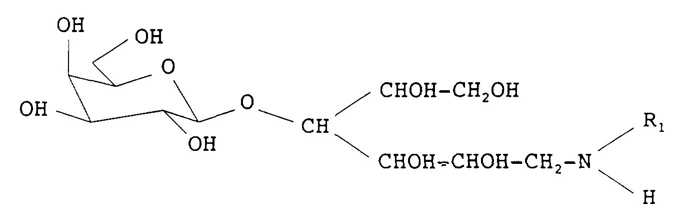 in der:
- R₁ für -(CH₂)ₙ-CH₃ oder -(CH₂)ₙ-COO⁻X⁺ steht, wobei
n bei 5 bis 15 liegt,
X⁺ für ein Kation steht, insbesondere ausgewählt aus H⁺, Na⁺, K⁺, NH₄⁺, Li⁺, Mg²⁺,
mit einem akylierenden Agens mit der Formel IV, R₂-CO-Z, reagieren läßt, in der Z insbesondere für 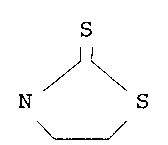 und R₂ für CH₃-(CH₂)ₘ-CO steht, wobei m bei 5 bis 16 liegt.

9. Verfahren zur Herstellung von Verbindungen, deren Struktur der Formel I entspricht: 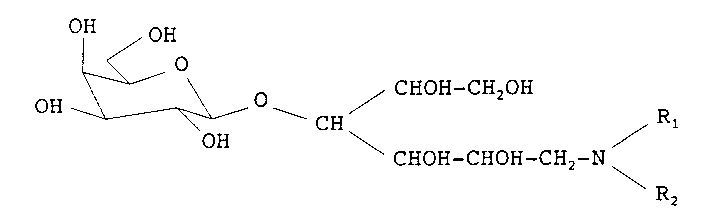 in der:
- R₁ für -(CH₂)ₙ-CH₃ oder -(CH₂)ₙ-COO⁻X⁺ steht, wobei
n bei 5 bis 15 liegt,
X⁺ für ein Kation steht, insbesondere ausgewählt aus H⁺, Na⁺, K⁺, NH₄⁺, Li⁺, Mg²⁺, und
- R₂ für CO-(CH₂)ₘ-CH₃ steht, wobei
m bei 5 bis 16 liegt,
dadurch gekennzeichnet, daß man in Gegenwart einer Base Lactose mit einer Aminosäure mit der Formel III, NH₂-(CH₂)ₙ-COOH, reagieren läßt, in der n wie oben definiert ist, anschließend das erhaltene N-Acyllactosylamin reduziert wird,
und man anschließend die so erhaltene Verbindung, die der Formel 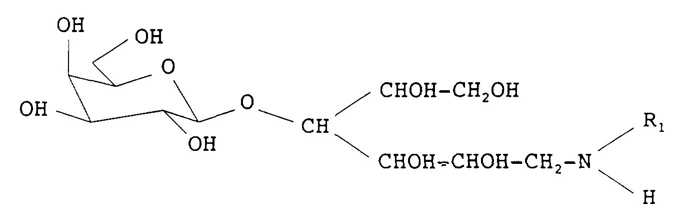 entspricht, in der:
- R₁ für -(CH₂)ₙ-CH₃ oder -(CH₂)ₙ-COO⁻X⁺ steht, wobei
n bei 5 bis 15 liegt,
X⁺ für ein Kation steht, insbesondere ausgewählt aus H⁺, Na⁺, K⁺, NH₄⁺, Li⁺, Mg²⁺,
mit einem akylierenden Agens mit der Formel IV, R₂-CO-Z, reagieren läßt, in der Z insbesondere für 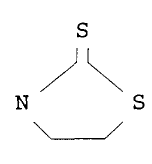 und R₂ für CH₃-(CH₂)ₘ-CO steht, wobei m bei 5 bis 16 liegt.

10. Verfahren nach Anspruch 9, dadurch gekennzeichnet, daß der Schritt der Reduktion mit NaBH₄ durchgeführt wird.

## Claims

1. Compounds whose structure corresponds to formula I: 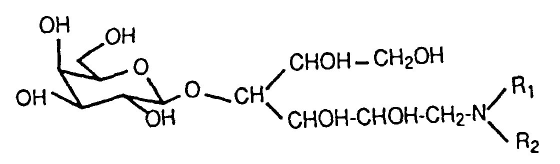 in which:
- R₁ represents -(CH₂)ₙ-CH₃ or -(CH₂)ₙ-COO⁻X⁺,
n being between 5 and 15,
X⁺ representing a cation chosen in particular from H⁺, Na⁺, K⁺, NH₄⁺, Li⁺ and Mg²⁺, and
- R₂ represents CO-(CH₂)ₘ-CH₃,
m being between 5 and 16.

2. Medicinal product comprising a compound whose structure corresponds to formula I: 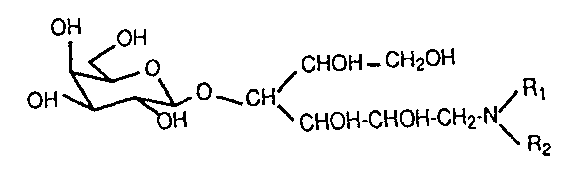 in which:
- R₁ represents -(CH₂)ₙ-CH₃ or -(CH₂)ₙ-COO⁻X⁺,
n being between 5 and 15,
X⁺ representing a cation chosen in particular from H⁺, Na⁺, K⁺, NH₄⁺, Li⁺ and Mg²⁺, and
- R₂ represents CO-(CH₂)ₘ-CH₃,
m being between 5 and 16,
or comprising a pharmaceutically acceptable salt of the said compound.

3. Use of a compound whose structure corresponds to formula I: 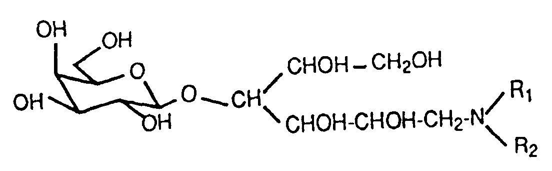 in which:
- R₁ represents-(CH₂)ₙ-CH₃ or -(CH₂)ₙ-COO⁻X⁺,
n being between 5 and 15,
X⁺ representing a cation chosen in particular from H⁺, Na⁺, K⁺, NH₄⁺, Li⁺ and Mg²⁺, and
- R₂ represents CO-(CH₂)ₘ-CH₃,
m being between 5 and 16,
or pharmaceutically acceptable salts of the said compound, to obtain a medicinal product intended for preventing or treating an infection with Aspergillus fumigatus.

4. Prophylactic or therapeutic medicinal composition comprising, as active principle, a compound whose structure corresponds to formula I: 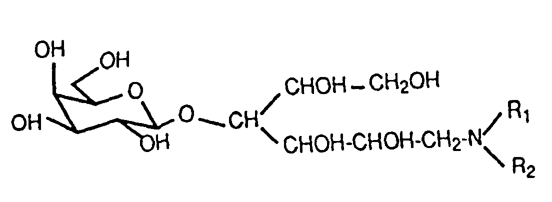 in which:
- R₁ represents -(CH₂)ₙ-CH₃ or -(CH₂)ₙ-COO⁻X⁺,
n being between 5 and 15,
X⁺ representing a cation chosen in particular from H⁺, Na⁺, K⁺, NH₄⁺, Li⁺ and Mg²⁺, and
- R₂ represents CO-(CH₂)ₘ-CH₃,
m being between 5 and 16,
or a pharmaceutically acceptable salt of the said compound, optionally combined with another active principle, and combined with a pharmaceutically acceptable vehicle.

5. Agent for preventing or treating an infection with HIV-I, comprising a compound whose structure corresponds to formula I: 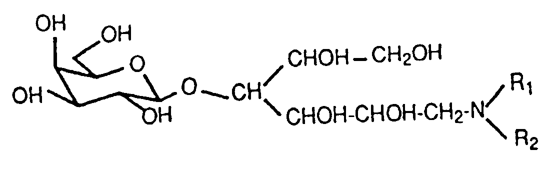 in which:
- R₁ represents-(CH₂)ₙ-CH₃ or -(CH₂)ₙ-COO⁻X⁺,
n being between 5 and 15,
X⁺ representing a cation chosen in particular from H⁺, Na⁺, K⁺, NH₄⁺, Li⁺ and Mg²⁺, and
- R₂ represents CO-(CH₂)ₘ-CH₃,
m being between 5 and 16,
or a pharmaceutically acceptable salt of the said compound.

6. Agent for preventing or treating an infection with Aspergillus fumigatus, comprising a compound whose structure corresponds to formula I: 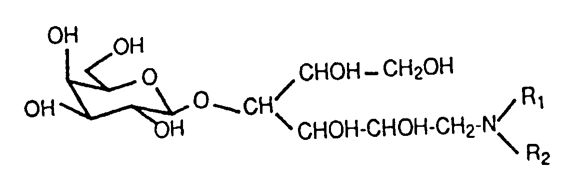 in which:
- R₁ represents -(CH₂)ₙ-CH₃ or -(CH₂)ₙ-COO⁻X⁺,
n being between 5 and 15,
X⁺ representing a cation chosen in particular from H⁺, Na⁺, K⁺, NH₄⁺, Li⁺ and Mg²⁺, and
- R₂ represents CO-(CH₂)ₘ-CH₃,
m being between 5 and 16,
or a pharmaceutically acceptable salt of the said compound.

7. Use of a compound whose structure corresponds to formula I: 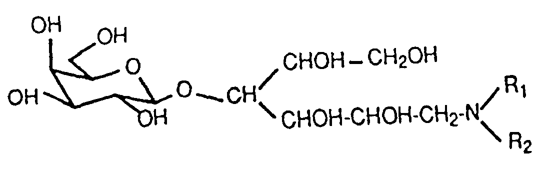 in which:
- R₁ represents-(CH₂)ₙ-CH₃ or -(CH₂)ₙ-COO⁻X⁺,
n being between 5 and 15,
X⁺ representing a cation chosen in particular from H⁺, Na⁺, K⁺, NH₄⁺, Li⁺ and Mg²⁺, and
- R₂ represents CO-(CH₂)ₘ-CH₃,
m being between 5 and 16,
or of a pharmaceutically acceptable salt, for preparing a medicinal product which is intended to prevent or treat an infection with HIV-I.

8. Process for preparing compounds whose structure corresponds to formula I: 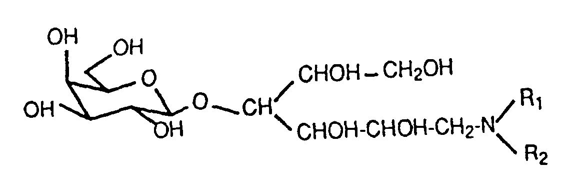 in which:
- R₁ represents -(CH₂)ₙ-CH₃ or -(CH₂)ₙ-COO⁻X⁺,
n being between 5 and 15,
X⁺ representing a cation chosen in particular from H⁺, Na⁺, K⁺, NH₄⁺, Li⁺ and Mg²⁺, and
- R₂ represents CO-(CH₂)ₘ-CH₃,
m being between 5 and 16,
characterized in that the compound corresponding to the formula 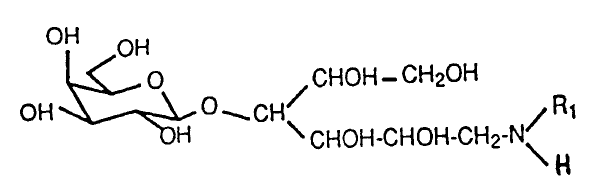 in which:
- R₁ represents-(CH₂)ₙ-CH₃ or -(CH₂)ₙ-COO⁻X⁺,
n being between 5 and 15,
X⁺ representing a cation chosen in particular from H⁺, Na⁺, K⁺, NH₄⁺, Li⁺ and Mg²⁺,
is reacted with an acylating agent of formula IV, R₂-CO-Z, in which Z represents in particular 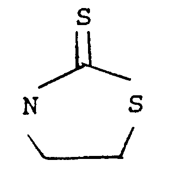 and R₂ represents CH₃-(CH₂)ₘ-CO, m being between 5 and 16.

9. Process for preparing compounds whose structure corresponds to formula I: 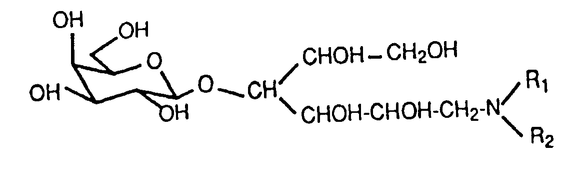 in which:
- R₁ represents -(CH₂)ₙ-CH₃ or -(CH₂)ₙ-COO⁻X⁺,
n being between 5 and 15,
X⁺ representing a cation chosen in particular from H⁺, Na⁺, K⁺, NH₄⁺, Li⁺ and Mg²⁺, and
- R₂ represents CO-(CH₂)ₘ-CH₃,
m being between 5 and 16,
characterized in that lactose is reacted with an amino acid of formula III, H₂N-(CH₂)ₙ-COOH in which n has the above definition, in the presence of a base, and the N-acyllactosylamine obtained is then reduced, after which, the compound thus obtained, corresponding to the formula 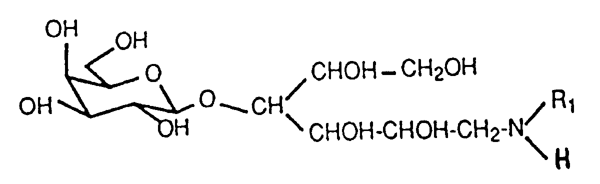 in which:
- R₁ represents-(CH₂)ₙ-CH₃ or -(CH₂)ₙ-COO⁻X⁺,
n being between 5 and 15,
X⁺ representing a cation chosen in particular from H⁺, Na⁺, K⁺, NH₄⁺, Li⁺ and Mg²⁺,
is reacted with an acylating agent of formula IV, R₂-CO-Z, in which Z represents in particular 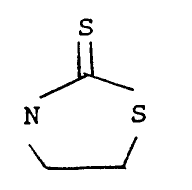 and R₂ represents CH₃-(CH₂)ₘ-CO, m being between 5 and 16.

10. Process according to Claim 9, characterized in that the reduction step is carried out with NaBH₄.
